# EUROPEAN PATENT APPLICATION

(11) **EP 3 995 152 A1**
(43) Date of publication of application: **11.05.2022**
(21) Application number: 21784542.9
(22) Date of filing: 23.03.2021
(51) Int. Cl.: A61K 47/61, A61K 47/60, A61K 47/69, A61K 31/704, A61K 31/454, A61K 31/517, A61P 35/00

(54) **HEPARIN NANO DRUG CARRIER SYSTEM FOR LOADING AMINO ANTI-TUMOR DRUG, AND PREPARATION METHOD THEREFOR**

(30) Priority: 09.04.2020 CN 202010275968
(71) Applicant: Nanjing King-Friend Biochemical Pharmaceutical Co., Ltd., Nanjing, Jiangsu 210061 (CN); Kindos Pharmaceuticals Co., Ltd., Chengdu, Sichuan 611731 (CN)
(72) Inventor: LI, Fangnian, Chengdu, Sichuan 611731 (CN); TIAN, Shane Xinxin, Chengdu, Sichuan 611731 (CN)
(74) Representative: v. Bezold & Partner Patentanwälte - PartG mbB
(86) International application number: PCT/CN2021/082331
(87) International publication number: WO 2021/203959

(57) **Abstract**

The present invention discloses a heparin nano drug carrier system loaded with an amino anti-tumor drug. The drug carrier system is a conjugate formed by loading the amino anti-tumor drug on a PEGylated heparin molecule. A natural polysaccharide heparin that is biodegradable and has good compatibility and high availability is used as a drug carrier, and by combining PEG modification and the amino anti-tumor drug, nanoparticles have a significantly enhanced anti-tumor therapeutic index and biological safety in in-vivo therapy when compared with free drugs.

## Description

### FIELD OF THE PRESENT DISCLOSURE

The present invention relates to the technical field of medicine, in particular to a heparin nano drug carrier system loaded with an amino anti-tumor drug and a preparation method thereof.

### BACKGROUND OF THE PRESENT DISCLOSURE

At present, with the rapid development of anti-tumor drug carriers, various new drug carrier systems are constantly emerging, and nano drug carrier systems based on macromolecular biomaterials emerge at the right moment. A multifunctional drug delivery system developed by combining macromolecular biomaterials with micro-molecular drugs through physical embedding or chemical bonding, can utilize an EPR effect of macromolecular carrier materials on solid tumors, and macromolecular carrier materials can selectively enrich drugs at tumor sites to achieve passive targeting. The controllable drug release of tumor micro-ambient intelligence response can be realized through specific physiological characteristics of the tumor sites compared with normal tissues, such as a pH value, a GSH level or the concentration of specific enzymes, so as to further improve the therapeutic effect of chemotherapy drugs and reduce toxicity and side effects. In addition, it is demonstrated that a nano drug carrier system can effectively improve the delivery of chemotherapy drugs into cells, by antagonizing or counteracting to active pumping-out of drugs by tumor cells, thereby reducing the drug resistance of tumor cells and improving the treatment level. The emergence of these new drug carrier systems is expected to realize the development and application of new anti-tumor drug formulations.

In recent years, macromolecular drug carrier systems such as dendrimers, polymers or polymer micelles, liposomes and so on are widely studied. Although a lot of researches on nano drug carriers have been reported, there is still a problem of poor biocompatibility of carriers. Some carriers will be cleared by a reticuloendothelial system (RES) after entering the body, while others can't pass through intracellular barriers such as cell membranes and nuclear membranes, so that they can hardly act on the target sites. Therefore, developing a drug carrier with targeting recognition to transport drugs to target tumor cells and tumor tissues to specifically kill cancer cells, is the primary objective in research of nano-preparations.

### SUMMARY OF THE PRESENT DISCLOSURE

Based on existing problems in the study of nano-preparations, the present invention provides a heparin nano drug carrier system loaded with an amino anti-tumor drug. A natural polysaccharide heparin that is biodegradable, and has good compatibility and high availability, is used as a drug carrier. By combining PEG modification and the amino anti-tumor drug, nanoparticles show a remarkably enhanced anti-tumor therapeutic index and biological safety during in vivo treatment when compared with free drugs.

In order to achieve the purpose of the present invention, the technical solution adopted by the present invention is as follows:

A heparin nano drug carrier system loaded with an amino anti-tumor drug is provided, wherein the drug carrier system is a conjugate formed by loading the amino anti-tumor drug onto a PEGylated heparin molecule; and a specific structure is as follows: 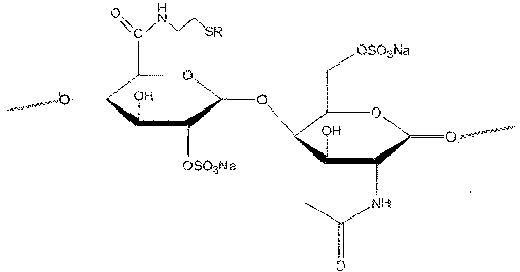
Where: R is a PEG group and a D group;
The PEG group is: 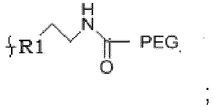
this group is an acyl group connected with a hydroxyl group at the end of PEG via an ester bond,
Where: R1 is 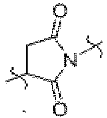 or -S-;
the structure of the D group is: 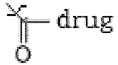 and this group is an acyl group connected with an amino group in the drug molecule via an amido bond.

The heparin nano drug carrier system of the present invention forms the nano-micelles loaded with the drug therein, so that metabolic kinetics of the drug can be changed, effect kinetics of the drug can be improved, a usage amount of the drug can be reduced, and the patient compliance can be improved. Targeting a target site accurately not only can increase a therapeutic effect, but also can reduce unnecessary toxic and side effects. The carrier heparin is of an endogenous structure, with a large safe dose; through combination with the drug molecule via chemical bonds, separation of the drug and ligands before use is avoided; and meanwhile, it improves targeting performance and safety by generation of specifical hydrolyzation effect after reaching the target site.

Polyethylene glycol (PEG) can enhance water solubility of materials and stability of plasma protein, reduce immunogenicity at the same time. PEG is used to modify the heparin nano-carrier, so as to reduce nonspecific cell phagocytosis of the nano-carrier by a mononuclear phagocyte system (MPS), and meanwhile, a circulating half-life of nano-particles can be adjusted.

The carrier is of a water-soluble heparin structure, which is transformed into a water-oil amphiphilic structure after the PEG is introduced; then a flexible ethyl sulfhydryl chain is introduced as a connecting bond, so that it not only reduces steric hindrance of a heparin sugar ring to subsequent conjugated compounds, but also is advantageous for adjusting a distribution state of the drug molecule in the micelle; and with a sulfhydryl as a binding site, types of the drug molecule to be conjugated can be increased.

The drug of the present invention is an anti-tumor drug containing a primary amino group or a secondary amino group.

A drug loading capacity of the nano drug carrier system of the present invention is preferably 4 wt%-15 wt%.

The polyethylene glycol (PEG) of the present invention is mPEG2000(MeO-PEG₂₀₀₀-OH).

The present invention further provides a preparation method of a heparin nano drug carrier system loaded with an amino anti-tumor drug, which comprises the following synthetic route:
(1) preparing a PEG derivative; 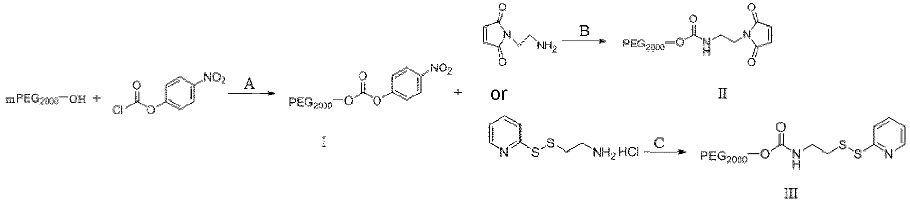
(2) preparing an intermediate A; 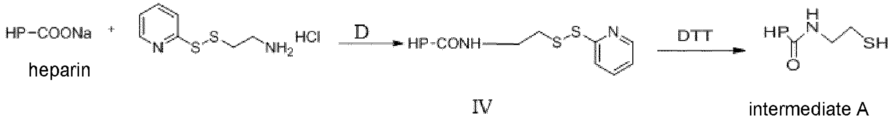
(3) reacting the intermediate A with the prepared PEG derivative to obtain a PEGylated heparin; and
(4) reacting the PEGylated heparin with 4-nitrophenyl chloroformate, and then adding the amino drug into the reaction system to obtain the heparin nano drug carrier system loaded with the drug.

In each of the reaction A and reaction B of step (1), a catalyst DIEA is added to neutralize HCI generated in the reaction and provide an alkaline environment to facilitate the reaction.

In the reaction C of step (1), catalysts DIEA and DMAP are added to neutralize HCI generated in the reaction and provide an alkaline environment to facilitate the reaction.

Preferably, in the reaction D, enoxaparin sodium is dissolved in a quinoline-8-sulfonic acid (MeS) buffer solution, and activated by adding 4-(4,6-dimethoxy-1,3,5-triazin-2-yl)-4-methyl morpholinium chloride (DMTMM), then S-(2-aminoethylthio)-2-thiopyridine (Py-SS-NH₂·HCl) that is dissolved in the MeS buffer solution is added dropwise to the system for reaction to obtain the intermediate A.

Further preferably, a preparation method of the MeS buffer comprises the following steps: weighing a quinoline-8-sulfonic acid and dissolving the quinoline-8-sulfonic acid in purified water, adding a sodium hydroxide solution dropwise to adjust the pH to 5.5, and making a metered volume to obtain the MeS buffer solution.

In the reaction of step (3), the intermediate A reacts with a polymer III, and triethylamine as a catalyst is added. The sulfhydryl group is weakly acidic, and the ionization degree of the sulfhydryl group and the activity of nucleophilic addition reaction increase with addition of triethylamine.

As for the heparin nano drug carrier system of the present invention, drugs containing primary or secondary amino groups, such as daunorubicin, lenalidomide, lapatinib, procarbazine, mitoxantrone, clofarabine, nelarabine, etc. can be connected therewith.

The present invention is advantageous in following aspects:
1. The nano drug carrier system of the present invention is of a heparin structure, and heparin is of an endogenous polysaccharide structure of natural organisms, which can be injected per se for medicinal use, thereby avoiding metabolic toxicity of synthetic/semi-synthetic materials; and PEG modification of the carrier can reduce the rigidity of the carrier, improve the amphiphilic performance and micellization ability of the carrier, and address the problem of poor biocompatibility of existing macromolecular drug carrier systems.
2. The drug carrier system uses flexible aliphatic chains to connect the drug molecule, which is more conducive to self-assembly of the system into the appropriate micelle in water, so that the drug molecule can be stably wrapped inside the micelle, which can prevent degradation due to external factors or unnecessary metabolic inactivation, and address the problem of poor stability of the existing macromolecular drug carrier systems; and using carbonate bonds to connect the carrier and the drug molecule can avoid separation of the drug molecule and the carrier under normal production/storage conditions, and after entering inside the body, the drug can be released at a designated site by catalysis of hydrolase, so as to realize targeting administration.
3. PEG is a polymer compound, and terminal OH activity thereof is not high, which is not conducive to subsequent reactions. According to the present invention, high-activity acyl chloride is used to react in a synthesis reaction, so that the intermediate I can be obtained in a high yield. P-nitrophenol is a good leaving group, which can be convenient for nucleophilic substitution in the subsequent reactions to connect target molecules.
4. Because the carboxyl group on the sugar ring of the heparin is influenced by steric hindrance of the sugar ring, the synthesis activity is low, which affects the yield. In the present invention, the flexible sulfhydryl aliphatic chain is introduced, which not only increases the reaction activity, but also helps to increase types of combination with drugs. The obtained intermediate A has 2-mercaptopyridine, which is an excellent leaving group, and will be replaced by nucleophilic substitution in case of more affinitive aliphatic sulfhydryl groups, which is convenient for obtaining of the PEGylated heparin through replacement of 2-mercaptopyridine in the PEG derivative reaction.
5. In the loading reaction of the amino drug, 4-nitrophenyl chloroformate is selected as a reaction raw material, and an active site thereof is acyl chloride, which can be quickly combined with the sulfhydryl group of the PEGylated heparin to form thioester, and the remaining 4-nitrophenyl is an excellent leaving group. When meeting the amino group with better nucleophilicity in the drug, amide is generated through nucleophilic addition, so that the drug can be loaded into the system smoothly.

### BRIEF DESCRIPTION OF THE DRAWINGS

Fig. 1 is a diagram showing morphological analysis of the appearance of a sample of a nano drug carrier system observed by a field emission transmission electron microscope (TEM), wherein a(1) is daunorubicin-Py-PEG-HP, and a(2) is daunorubicin-Mal-PEG-HP; b(1) is lenalidomide-Py-PEG-HP, and b(2) is lenalidomide-Mal-PEG-HP; and c(1) is lapatinib-Py-PEG-HP, and c(2) is lapatinib-Mal-PEG-HP.
Fig. 2 is a diagram showing in vitro hemolysis experimental results of a nano drug carrier system of the present invention, wherein a(1) is daunorubicin of Py-PEG-HP, a(1) is daunorubicin-Py-PEG-HP, and a(2) is daunorubicin-Mal-PEG-HP; b(1) is lenalidomide-Py-PEG-HP, and b(2) is lenalidomide-Mal-PEG-HP; and c(1) is lapatinib-Py-PEG-HP, and c(2) is lapatinib-Mal-PEG-HP.
Fig. 3 is a column chart showing in vivo experimental results of breast cancer cells under a daunorubicin heparin nano drug carrier system.
Fig. 4 is a column chart showing in vivo experimental results of breast cancer cells under a nano drug carrier system of lenalidomide and lapatinib.

### DESCRIPTION OF THE EMBODIMENTS

In order to explain the target technical solution of the present invention more clearly in detail, the present invention will be further described by related embodiments below. The following embodiments only specifically illustrate implementation methods of the present invention, and are not intended for limiting the scope of the present invention.

### Embodiment 1

Daunorubicin is loaded on a PEGylated heparin molecules, with the following structure:

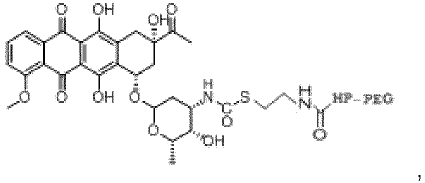

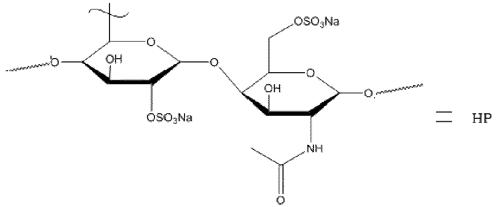

Where:
PEG-HP represents a PEGylated heparin polymer, with the structure as follows: 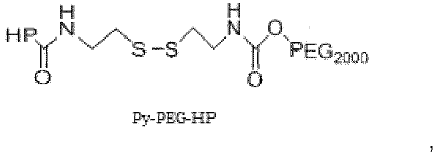
or 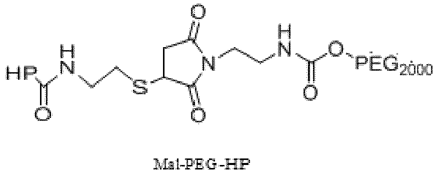
Where: the acyl group is connected with the hydroxyl group at the end of PEG via an ester bond.

### Embodiment 2

Lenalidomide is loaded on a PEGylated heparin molecule, with the following structure: 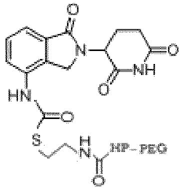
PEG-HP represents a PEGylated heparin polymer with the structure as follows: 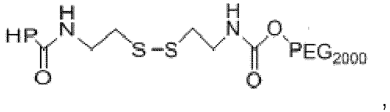
or 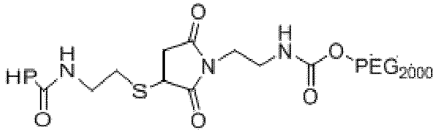
Where: the acyl group is connected with the hydroxyl group at the end of PEG via an ester bond.

### Embodiment 3

Lapatinib is loaded on a PEGylated heparin molecule, with the following structure: 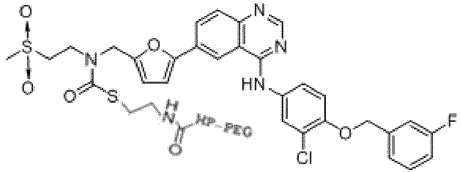
PEG-HP represents a PEGylated heparin polymer with the structure as follows: 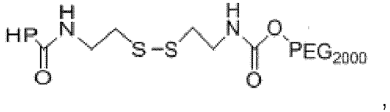 or 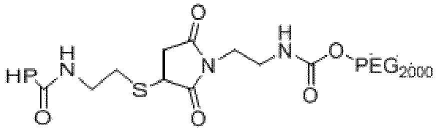
Where: the acyl group is connected with the hydroxyl group at the end of PEG via an ester bond.

### Embodiment 4

A preparation method of a heparin nano drug carrier system loaded with an amino anti-tumor drug comprises the following synthetic route:
(1) a PEG derivative is prepared; 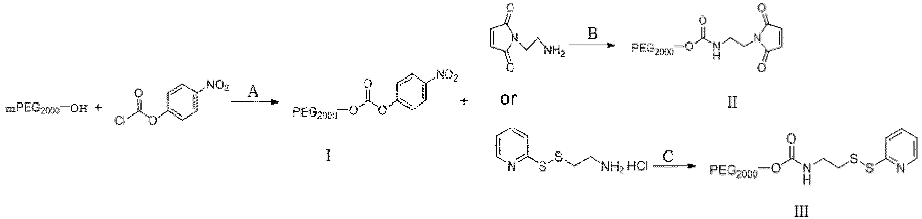
(2) an intermediate A is prepared; 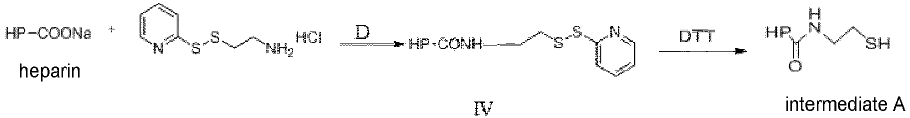 Acyl groups of some units in the heparin react with 4-(4,6-dimethoxy-1,3,5-triazin-2-yl)-4-methyl morpholinium chloride (DMTMM) to obtain a polymer IV, which is further desulfurized, to obtain the intermediate A. The specific structural formula of intermediate A is as follows: 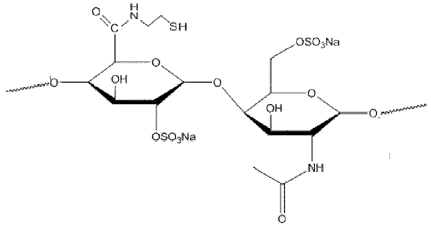
(3) the intermediate A reacts with the prepared PEG derivative to obtain a PEGylated heparin (namely, the PEGylated intermediate A);
(4) the PEGylated heparin reacts with 4-nitrophenyl chloroformate, -SH in the polymer unit of the intermediate A, which is not replaced by a PEG group reacts with acyl chloride of 4-nitrophenyl chloroformate to obtain a -OCOS- bond, and then the amino drug is added into the reaction system to generate -NCOS- and thus obtain the PEGylated heparin nano drug carrier system loaded with the drug.

### Embodiment 5

The embodiment is based on Embodiment 4:
In the reaction A and reaction B of step (1), a catalyst DIEA is added to neutralize HCI generated in the reaction and the reaction is facilitated by provision of an alkaline environment.

In the reaction D, enoxaparin sodium is dissolved in a quinoline-8-sulfonic acid (MeS) buffer solution, and activated by adding 4-(4,6-dimethoxy-1,3,5-triazin-2-yl)-4-methyl morpholinium chloride (DMTMM), then S-(2-aminoethylthio)-2-thiopyridine (Py-SS-NH₂·HCl) that is dissolved in the MeS buffer solution is added to the system dropwise for reaction to obtain the intermediate A.

In the reaction of step (3), the intermediate A reacts with a polymer III, and triethylamine as a catalyst is added. The sulfhydryl group is weakly acidic, and the ionization degree of the sulfhydryl group and the activity of nucleophilic addition reaction increase by addition of triethylamine.

### Embodiment 6

Based on Embodiment 4, this embodiment is carried out as follows.

In the reaction A of step (1), a catalyst DIEA is added to neutralize HCI generated in the reaction and provide an alkaline environment to facilitate the reaction.

In the reaction C of step (1), catalysts DIEA and DMAP are added to neutralize HCI generated in the reaction and provide an alkaline environment to facilitate the reaction.

In the reaction D, enoxaparin sodium is dissolved in a quinoline-8-sulfonic acid (MeS) buffer solution, and activated by adding 4-(4,6-dimethoxy-1,3,5-triazin-2-yl)-4-methyl morpholinium chloride (DMTMM) thereto, then S-(2-aminoethylthio)-2-thiopyridine (Py-SS-NH₂·HCl) that is dissolved in the MeS buffer solution is added to the system dropwise for reaction to obtain the intermediate A.

A preparation method of the MeS buffer comprises the following steps: a quinoline-8-sulfonic acid is weighed and dissolved in purified water, a sodium hydroxide solution is added dropwise to adjust the pH to 5.5, and a metered volume is made to obtain the MeS buffer solution.

In the reaction of step (3), the intermediate A reacts with a polymer III, and triethylamine as a catalyst is added. The sulfhydryl group is weakly acidic, and the ionization degree of the sulfhydryl group and the activity of nucleophilic addition reaction are increased by addition of triethylamine.

### Embodiment 7

### Synthesis of a PEG derivative

### Reaction A:

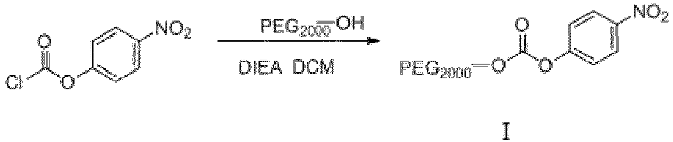

20 g of mPEG2000 is dissolved in 100mL of DCM, with addition of 8 ml of diisopropylethylamine (DIEA) thereto dropwise in an ice bath. The system is colorless and transparent. 8 g of 4-nitrophenyl chloroformate is dissolved in 50 mL of DCM, and added to the above solution dropwise under the ice bath. After dropping, the system slowly rises to a room temperature. At this time, there is no obvious change in the system, and the reaction is carried out overnight.

Separation and purification: the system is bright yellow and slightly turbid, filtered, and then spun to dry to obtain a yellow viscous liquid. 200 mL of ethyl acetate (EA) is first added into the system and stirred vigorously at a room temperature, the yellow liquid gradually turns into a white solid which is dispersed in the system, the liquid turns into bright yellow, and it added 100 mL of Et₂O dropwise while stirring, pulped at the room temperature for 0.5 h, and stirred to obtain a white solid. The white solid is transferred to a beaker, 200 mL of EA is first added while stirring, then 100 mL of Et₂O is added dropwise after 15 min, pulped for 0.5 h, suction filtration is performed to obtain a white solid, and the solid is dried under a reduced pressure to obtain 16.1 g of the white solid (polymer I).

### Reaction B:

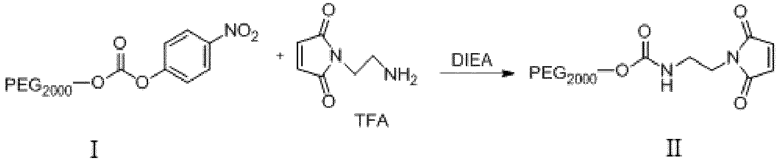

0.65 g of DIEA is weighed and put into a round-bottom flask, a DCM solution is added under an ice bath, then 0.47 g of Maleic-NH₂·TFA(N-(2-aminoethyl)maleimide trifluoroaceta) is added with stirring for 0.5 h, finally 4.3 g of the polymer I is added dropwise into the DCM solution, and it is kept overnight at ambient temperature after the completion of addition. The next day, suction filtration is performed, and the filtrate is spun to dry to obtain a yellow oily substance. Pulping is performed twice with 300 mL of a liquid with EA/tert-methyl ether=2/1, and the product is dried under a reduced pressure to obtain 3.9 g of a white solid (polymer II).

### Embodiment 8

### Synthesis of a PEG derivative

Preparation of the polymer I is the same as Embodiment 7.

### Reaction C:

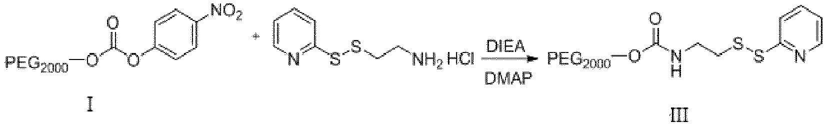

0.65 g of DIEA and 0.12 g of DMAP(4-dimethylaminopyridine) are weighed and put into a round-bottom flask, a DCM solution is added under an ice bath, then 0.45 g of S-(2-aminoethylthio)-2-thiopyridine (Py-SS-NH₂) is added for stirring of 0.5 h, finally the DCM solution with 4.3 g of the polymer I is added dropwise, and after that, it is kept overnight at ambient temperature. The next day, suction filtration is performed, and the filtrate is spun to dry to obtain a yellow oily substance. Pulping is performed twice with 300 mL of a liquid with EA/tert-methyl ether=2/1, and the product is dried under a reduced pressure to obtain 4.2 g of a white solid (polymer III).

### Embodiment 9

### Synthesis of an intermediate A

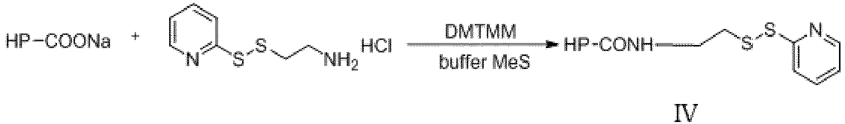

2.88g of enoxaparin sodium (HPCOONa) is dissolved in 10 mL of a MeS buffer solution, and activated for 10 min by adding 4.14 g of 4-(4,6-dimethoxy-1,3,5-triazin-2-yl)-4-methyl morpholinium chloride (DMTMM), then 3.34 g of Py-SS-NH₂·HCl that is dissolved in the 10 mL of MeS buffer solution is added to the system dropwise for reaction of 24 h, thereafter, it is dialyzed for 3 days and lyophilized, to yield 1.5 g of a product (polymer IV).

Preparation of the MeS buffer solution: 2 g of sodium hydroxide is weighed, dissolved in 20 mL of purified water, cooled for later use. 9.8 g of quinoline-8-sulfonic acid is weighed and dissolved in 250 mL of purified water, with addition of the sodium hydroxide solution dropwise to adjust the pH to 5.5, and a metered volume is made to 500 mL.

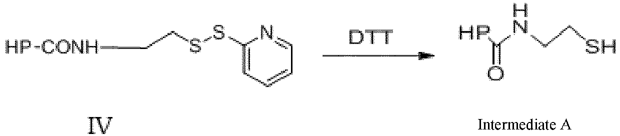

1.5 g of the polymer IV is dissolved in water, 1.5 g of dithiothreitol (DTT) is added at a room temperature, reaction is carried out overnight, and it is dialyzed with a semipermeable membrane of 1 KDo for three days since the next day, and lyophilized, to obtain 1.1 g of a white solid (intermediate A).

### Embodiment 10

### Synthesis of Py-PEG-HP

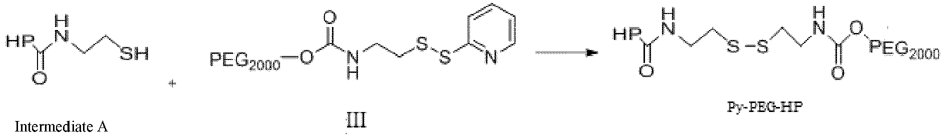

3.1 g of the intermediate A is dissolved in water, 1.2 g of the polymer III is added at 35°C, reaction is performed overnight, and it is dialyzed with a semipermeable membrane of 1 KDo for two days since the next day, and lyophilized to obtain 3.7 g of a white solid (Py-PEG-HP). HNMR (D₂O+D-DMSO): 2.8-3.0 (SCH₂CH₂N), 3.0-3.3 (heparin sodium sugar ring hydrocarbon), 3.4-3.7 (methylene hydrogen in PEG), and 3.8(CH₃O-).

### Embodiment 11

### Synthesis of Mal-PEG-HP

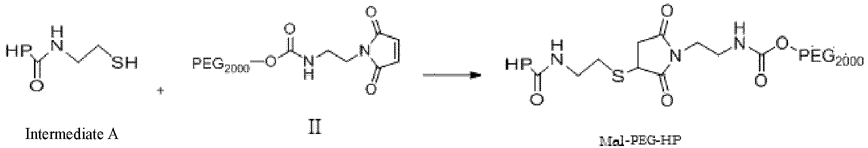

3.1 g of the intermediate A is dissolved in water, 1.3 g of the polymer II is added at a room temperature, and then 0.05 g of triethylamine is added as a catalyst. Reaction is performed overnight, and it is dialyzed with a semipermeable membrane of 1 KDo for two days since the next day, and lyophilized, to obtain 3.9 g of a white solid (Mal-PEG-HP). HNMR (D₂O+D-DMSO): 2.7 (CO-CH₂), 2.9 (CH₂ connected to NH) 3.0-3.3 (heparin sodium sugar ring hydrocarbon), 3.4-3.7 (methylene hydrogen in PEG), 3.8(CH₃O-), and 3.9(S-CH₂-CO).

### Embodiment 12

### Synthesis of a conjugate of daunorubicin and Py-PEG-HP

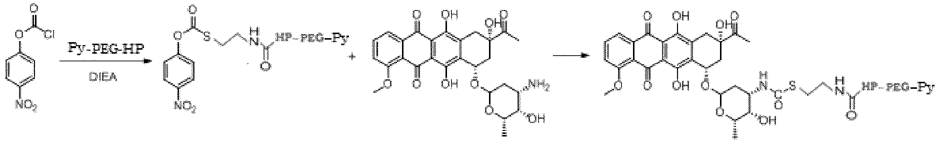

0.3 g of Py-PEG-HP (PEGylated intermediate A) is dissolved in 20 ml of DMSO, 0.13 g of diisopropylethylamine (DIEA) is added under an ice bath, and the system is colorless and slightly turbid. Then, 0.21 g of 4-nitrophenyl chloroformate is added into the above solution. After addition, the system slowly rises to a room temperature. After the reaction is performed for 12 hours, 0.53 g of daunorubicin is added into the system and the reaction is kept at 35°C for 24 hours. Dialysis with a semipermeable membrane of 3.5 KDo is performed on the system for three days, and the liquid is filtrated and lyophilized, to obtain 0.38 g of a red solid. HNMR (D₂O+D-DMSO): 1.2 (monomethyl hydrogen on the API ring), 1.9-2.4 (aliphatic carbocyclic hydrogen of API), 2.5 (carbonyl a-methyl in API), 2.8-3.0 (SCH₂CH₂N), 3.0-3.3 (hydrocarbon in heparin sodium sugar ring), 3.4-3.7 (methylene hydrogen in PEG), 3.8 (CH₃O-), 4.5 (hydrogen on two ends of oxygen of API), and 7.5-7.9 (benzene ring hydrogen).

Using UV(SP-1920UV, Shanghai Spectrum Instruments Co., Ltd.), the drug loading capacity of daunorubicin-Py-PEG-HP is measured as 8.2%.

### Embodiment 13

### Synthesis of a conjugate of daunorubicin and Mal-PEG-HP

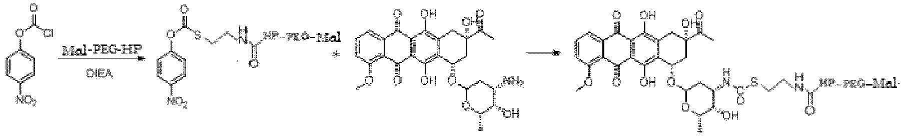

0.3 g of Mal-PEG-HP (PEGylated intermediate A) is dissolved in 20 ml of DMSO, 0.13 g of diisopropylethylamine (DIEA) is added under an ice bath, and the system is colorless and slightly turbid. Then, 0.21 g of 4-nitrophenyl chloroformate is added into the above solution. After addition, the system slowly rises to room temperature. After the reaction is performed for 12 hours, 0.53 g of daunorubicin is added into the system and the reaction is kept at 35°C for 24 hours. Dialysis with a semipermeable membrane of 3.5 KDo is performed on the system for three days, and the liquid is filtrated and lyophilized, to obtain 0.42 g of a red solid. HNMR (D₂O+D-DMSO): 1.2 (monomethyl hydrogen on the API ring), 1.9-2.4 (aliphatic carbocyclic hydrogen of API), 2.5 (carbonyl a-methyl in API), 2.7 (CO-CH₂), 2.9 (CH₂ connected to NH), 3.0-3.3 (hydrocarbon in heparin sodium sugar ring), 3.4-3.7 (methylene hydrogen in PEG), 3.8 (CH₃O-), 3.9 (S-CH₂-CO), 4.5 (hydrogen on two ends of oxygen of API), and 7.5-7.9 (benzene ring hydrogen).

Using UV(SP-1920UV, Shanghai Spectrum Instruments Co., Ltd.), the drug loading capacity of daunorubicin-Mal-PEG-HP is measured as 6.8%.

### Embodiment 14

### Synthesis of a conjugate of lenalidomide and Py-PEG-HP

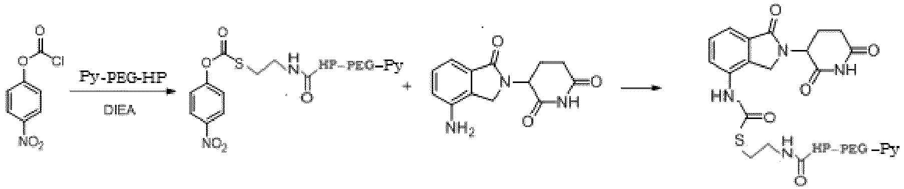

0.3 g of Py-PEG-HP (PEGylated intermediate A) is dissolved in 20 ml of DMSO, 0.13 g of diisopropylethylamine (DIEA) is added under an ice bath, and the system is colorless and slightly turbid. Then, 0.21 g of 4-nitrophenyl chloroformate is added into the above solution. After addition, the system slowly rises to a room temperature. After the reaction is performed for 12 hours, 0.26 g of lenalidomide is added into the system and the reaction is kept at the room temperature for 24 hours. Dialysis with a semipermeable membrane of 3.5 KDo is performed on the system for three days, and the liquid is filtrated and lyophilized, to obtain 0.34 g of a light yellow solid. HNMR (D₂O+D-DMSO): 2.0-2.4 (ethyl between nitrogen-carbonyl of API), 2.5 (carbonyl a-methyl in API), 2.8-3.0 (SCH₂CH₂N), 3.0-3.3 (hydrocarbon in heparin sodium sugar ring), 3.4-3.7 (methylene hydrogen in PEG), 3.8 (CH₃O-), 4.4 (hydrogen on two ends of oxygen of API), and 6.8-7.4 (benzene ring hydrogen).

Using UV(SP-1920UV, Shanghai Spectrum Instruments Co., Ltd.), the drug loading capacity of lenalidomide-Py-PEG-HP is measured as 11.8%.

### Embodiment 15

### Synthesis of a conjugate of lenalidomide and Mal-PEG-HP

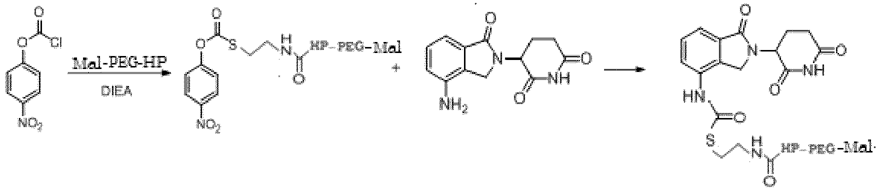

0.3 g of Mal-PEG-HP (PEGylated intermediate A) is dissolved in 20 ml of DMSO, 0.13 g of diisopropylethylamine (DIEA) is added under an ice bath, and the system is colorless and slightly turbid. Then, 0.21 g of 4-nitrophenyl chloroformate is added into the above solution. After addition, the system slowly rises to a room temperature. After the reaction is performed for 12 hours, 0.26 g of lenalidomide is added into the system and the reaction is kept at the room temperature for 24 hours. Dialysis with a semipermeable membrane of 3.5 KDo is performed on the system for three days, and the liquid is filtrated and lyophilized, to obtain 0.38 g of a light yellow solid. HNMR (D₂O+D-DMSO): 2.0-2.4 (ethyl between nitrogen-carbonyl of AP API), 2.7 (CO-CH₂), 2.9 (CH₂ connected to NH), 3.0-3.3 (hydrocarbon in heparin sodium sugar ring), 3.4-3.7 (methylene hydrogen in PEG), 3.8 (CH₃O-), 3.9 (S-CH₂-CO), 4.4 (hydrogen on two ends of oxygen of API), and 6.8-7.4 (benzene ring hydrogen).

Using UV(SP-1920UV, Shanghai Spectrum Instruments Co., Ltd.), the drug loading capacity of lenalidomide-Mal-PEG-HP is measured as 13.1%.

### Embodiment 16

### Synthesis of a conjugate of lapatinib and Py-PEG-HP

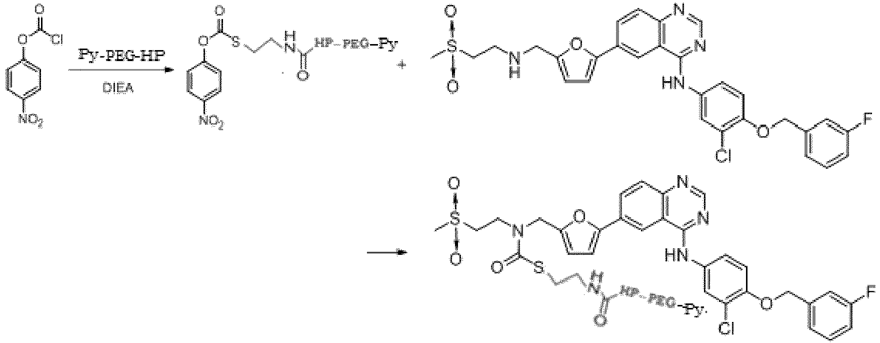

0.3 g of Py-PEG-HP (PEGylated intermediate A) is dissolved in 20 ml of DMSO, 0.13 g of diisopropylethylamine (DIEA) is added in an ice bath, and the system is colorless and slightly turbid. Then, 0.21 g of 4-nitrophenyl chloroformate is added into the above solution. After addition, the system slowly rises to a room temperature. After the reaction is performed for 12 hours, 0.58 g of lapatinib is added into the system and the reaction is kept at 50°C for 24 hours. Dialysis with a semipermeable membrane of 3.5 KDo is performed on the system for three days, and the liquid is filtrated and lyophilized, to obtain 0.34 g of a light yellow solid. HNMR (D₂O+D-DMSO): 2.7 (sulfuryl ortho methyl of API), 2.5 (carbonyl a-methyl in API), 2.8-3.0 (SCH₂CH₂N), 3.0-3.3 (hydrocarbon in heparin sodium sugar ring), 3.4-3.7 (methylene hydrogen in PEG), 3.8 (CH₃O-), 4.4 (hydrogen on two ends of oxygen of API), 5.2 (O-CH₂-Ph), 6.8-7.5 (furan ring, chlorine-substituted ring and fluoro-substituted ring) and 8.0-8.4 (benzopyrimidine ring).

Using UV(SP-1920UV, Shanghai Spectrum Instruments Co., Ltd.), the drug loading capacity of lapatinib-Py-PEG-HP is measured as 9.2%.

### Embodiment 17

### Synthesis of a conjugate of lapatinib and Mal-PEG-HP

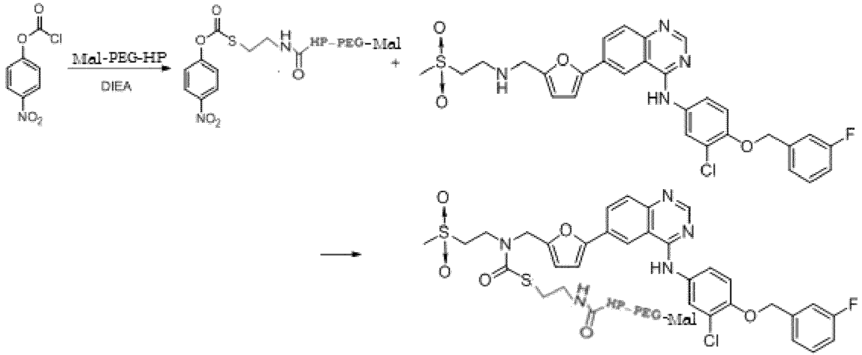

0.3 g of Mal-PEG-HP (PEGylated intermediate A) is dissolved in 20 ml of DMSO, 0.13 g of diisopropylethylamine (DIEA) is added in an ice bath, and the system is colorless and slightly turbid. Then, 0.21 g of 4-nitrophenyl chloroformate is added into the above solution. After addition, the system slowly rises to a room temperature. After the reaction is performed for 12 hours, 0.58 g of lapatinib is added into the system and the reaction is kept at 50°C for 24 hours. Dialysis with a semipermeable membrane of 3.5 KDo is performed on the system for three days, and the liquid is filtrated and lyophilized, to obtain 0.41 g of a light yellow solid. HNMR (D₂O+D-DMSO): 2.7 (sulfuryl ortho methyl of API), 2.7 (CO-CH₂), 2.9 (CH₂ connected to NH), 3.0-3.3 (hydrocarbon in heparin sodium sugar ring), 3.4-3.7 (methylene hydrogen in PEG), 3.8 (CH₃O-), 3.9 (S-CH₂-CO), 4.4 (hydrogen on two ends of oxygen of API), 5.2 (O-CH₂-Ph), 6.8-7.5 (benzene ring hydrogen), 6.8-7.5 (furan ring, chlorine-substituted ring and fluoro-substituted ring) and 8.0-8.4 (benzopyrimidine ring).

Using UV(SP-1920UV, Shanghai Spectrum Instruments Co., Ltd.), the drug loading capacity of lapatinib-Mal-PEG-HP is measured as 10.8%.

The heparin nano drug carrier system of the present invention not only can load daunorubicin, lenalidomide and lapatinib, but can also load other drugs containing primary or secondary amino groups such as procarbazine, mitoxantrone, clofarabine, nelarabine, and synthesis methods are the same.

Raw material sources:
4-dimethylaminopyridine (DMAP) Chengdu Kelong Chemical Reagent Factory
4-(4,6-dimethoxy-1,3,5-triazin-2-yl)-4-methyl morpholinium chloride (DMTMM) J&K Scientific Co., Ltd.
4-nitrophenyl chloroformate Tianjin HEOWNS Biochemical Technology Co., Ltd.
DL-dithiothreitol (DTT) Aladdin Biochemical Technology Co., Ltd.
Quinoline-8-sulfonic acid (MeS) Aladdin Biochemical Technology Co., Ltd.
S-(2-aminoethylthio)-2-thiopyridine Shanghai BioChemPartner Co., Ltd.
N-(2-aminoethyl)maleimide trifluoroacetate Aladdin Biochemical Technology Co., Ltd.

### Morphological analysis

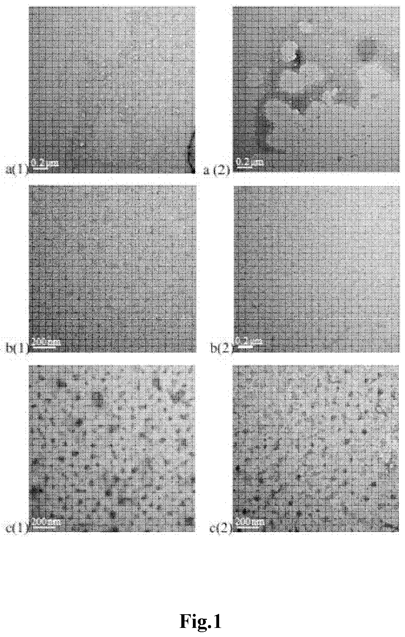

Upon the observation of the appearance of the samples by the field emission transmission electron microscope (TEM) (American FEI Company, Tecnai G2 F20 S-TWIN, Analysis and Testing Center, Sichuan University), the morphological analysis of heparin nano drug carrier systems of daunorubicin, lenalidomide and lapatinib is shown in Fig. 1. As can be seen from the analysis data of electron microscope, the sample is nearly a spherical nanoparticle with a size of about 80-100nm.

### In vitro hemolysis test

Blood biocompatibility is one of the important items to evaluate the biological safety of intravenous injection of MRI contrast agents. 2 mL of fresh blood from healthy BALB/c mice is collected in a heparin tube, centrifuged at a rotating speed of 3000 g for 5 min, and red blood cells (erythrocyte) are isolated at 4°C. The resultant red blood cells are suspended in 20% PBS. 6 heparin nano drug carrier systems of daunorubicin, lenalidomide and lapatinib are added to the above-mentioned red blood cell solution (50 µL) respectively, with settings of the polymer concentrations as 0.5 and 4 mg/mL. Incubation is performed at 37°C for 24 h. Then, the above-mentioned red blood cell suspension is centrifuged for 3 min at a rotating speed of 3000 g so as to get supernatant, which is detected for the absorbance at 540 nm by a multifunctional microplate reader (BioTek, EON). In the experiment, PBS was used as negative control and purified water was used as positive control.

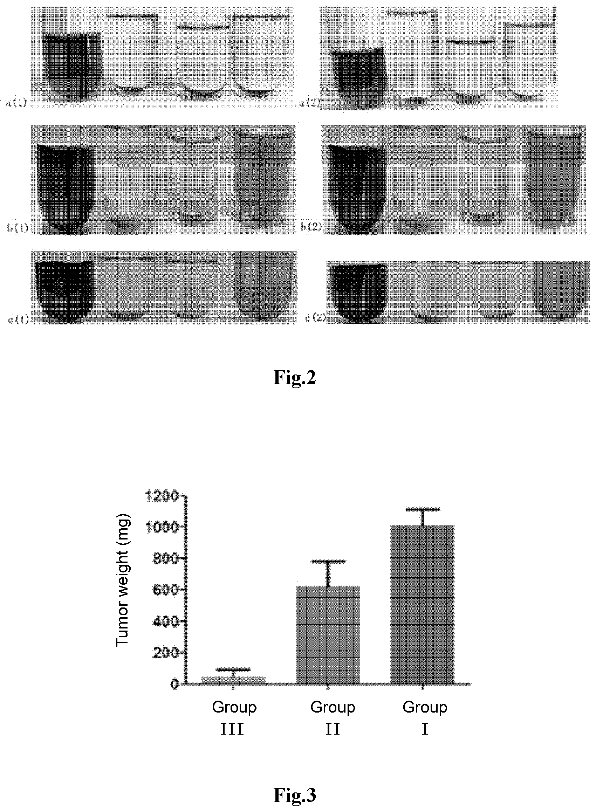

The results are shown in Fig. 2 (from left to right: blood sample, PBS, 0.5 mg/mL, 4 mg/mL). At 37°C, the red blood cells of normal mice are incubated with each nano drug carrier system for 24 hours. After centrifugation, all of the supernatants do not appear distinct red, it is demonstrated that all the nano-micelles do not cause hemolysis and have good biocompatibility, due to no distinct red of the supernatant.

### Evaluation of anti-tumor effects

### In vivo experiment of breast cancer cells

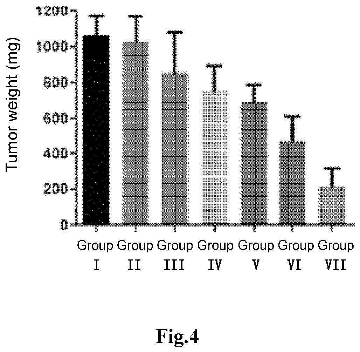

Anti-tumor effects of nanoparticles in vivo are studied in BALB/c mice with 4T1 breast cancer xenograft tumors. Free daunorubicin (Group I), daunorubicin-Py-PEG-HP (Group II) and daunorubicin-Mal-PEG-HP (Group III) are administered via caudal veins at a dose of 3 mg/kg, for once every three days and in total 4 times, with the free daunorubicin group (Group I) as the control. As shown in Fig. 4, tumor development is detected on the third day after administration. Considering that tumors will continue to develop normally, free daunorubicin shows insignificant anti-tumor activity, while both groups of nanoparticles show high anti-cancer effects. Especially, the tumor volumes of mice in group III obviously shrink on the third day, and the trend continues to the end of treatment. These results indicate that nanoparticles have a better anti-tumor effect than free daunorubicin in vivo.

The anti-tumor effect of nanoparticles in vivo is based on investigation of a xenotransplantation 4T1 tumor model established in the BALB/c mice. Each of groups represents normal saline (Group I), free lenalidomide (Group II), lenalidomide-Py-PEG-HP (Group III), lapatinib-Mal-PEG-HP and lenalidomide-Mal-PEG-HP (Group IV), free lapatinib (Group V), lapatinib-Py-PEG-HP (Group VI) and lapatinib-Mal-PEG-HP (Group VII) respectively, wherein they are administrated via caudal veins at a dosage of 4 mg/kg, once every two days, with a total of 5 times. As shown in the diagram below, the tumor development is detected on the second day after administration. With the normal saline group as control, it found that all groups have the anti-tumor activity. Based on grouped investigation, the tumor cells in the nanoparticle treatment group all contracted obviously, and the therapeutic effect thereof is better than that of the corresponding free drug groups. These results demonstrate that nanoparticles have a better anti-tumor effect than free drugs in vivo.

### Uptake experiment of leukemia cells

An uptake experiment of free daunorubicin, daunorubicin-Py-PEG-HP and daunorubicin-Mal-PEG-HP with acute promyelocytic leukemia (HL-60) cells: the concentration of HL-60 cells is adjusted to 1×10⁵/ml, and the cells are added into a 24-well plate with 1 ml per well; a certain concentration of pure DNR solution and a certain concentration of DNR-NP suspension are added into a culture plate, and cultured for a certain time, then the cells are taken out at five time points (30 min, 60 min, 90 min, 120 min and 180 min) respectively; centrifugation is performed at 4°C under 1000 rpm for 5 min, and the cells are collected, and then washed twice using a RPMI1640 medium added with serum; the cells are lysed with 1.5 ml of 50 µg/ml protease K and a 1% SDS solution, with continuous shaking performed at 37°C for 12 hours, and a cell extract is collected; and the drug content in the cells is calculated by a fluorescence intensity standard curve.

The results show that, by the HL-60 cells, uptake efficiency of daunorubicin-Py-PEG-HP is 43%, and uptake efficiency of daunorubicin-Mal-PEG-HP is 52%, while uptake efficiency of free daunorubicin is only 16%.

### In vivo experiment of multiple myeloma

Thirty 6-week-old mice are divided into three groups, which are administrated with free lenalidomide (Group I), lenalidomide-Py-PEG-HP (Group II) and lenalidomide-Mal-PEG-HP (Group III) respectively. Male and female animals in each group are halved, and there is no statistically significant difference in body weight among animals in each group. The dosage of free lenalidomide group is 0.1 ml/kg body weight/day, and the dosage of lenalidomide-Py-PEG-HP group and lenalidomide-Mal-PEG-HP group is 20 mg/kg (body weight/day).

One week after injection of 1,000,000 5T33 multiple myeloma cells into the tail veins of mice, drugs are administered subcutaneously according to the above scheme, for three times a week. During the administration period, the animals are weighed every day, and the dosage on that day is determined according to the weight, and the animals are continuously administered until death of the mice. The venous blood of mice is collected and stored every week, and the time of death of mice is recorded.

Experimental results demonstrate that subcutaneous injection of lenalidomide-Py-PEG-HP and lenalidomide-Mal-PEG-HP could significantly prolong the survival time of mice and reduce the tumor load in mice serum (mouse LGg2b concentration). According to the statistical test, there is significant difference in the survival time of mice when free lenalidomide group is compared with heparin nano drug carrier system (P<0.05), as shown in Table 1. There is significant difference in the tumor load in serum of mice when the free lenalidomide group is compared with heparin nano drug carrier system (P<0.05), as shown in Table 2.

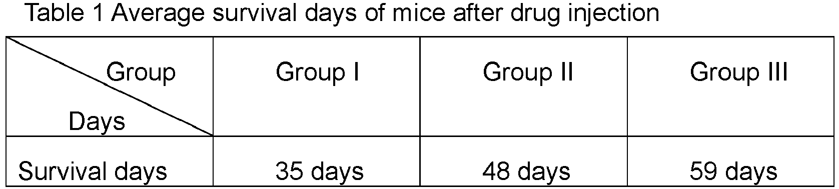

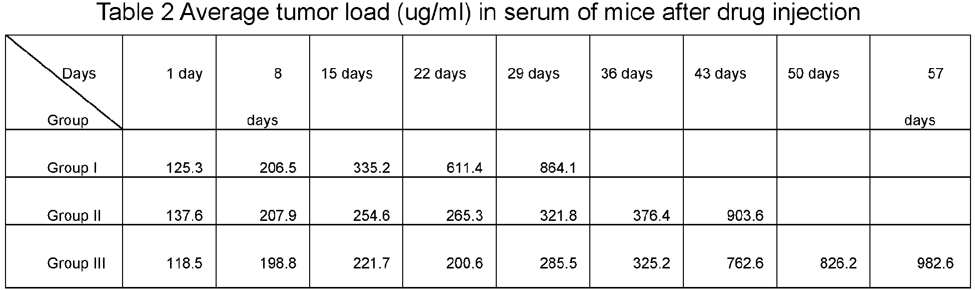

### In vivo toxicity evaluation

Healthy female BALB/c mice aged 6 to 8 weeks (weighing about 20±2 g) are randomly divided into 6 groups with 7 mice in each group, and each mouse is labeled. Then, drugs (200 µL) are injected into mice via tail vein injection, which are 6 kinds of heparin nanoparticles composed of daunorubicin, lenalidomide and lapatinib synthesized by the present invention and normal saline as the control group, wherein the injection dosage is uniformly 20 mg/kg, and the injection is carried out once a day for 10 times totally. Weights of mice are recorded every two days and behaviors of the mice are observed, with the first-day weight set as 100%. 19 days later, the mice are euthanized and blood of the mice is collected for analysis.

During the overall treatment study, it led to generally good tolerance with respect to repeated injections of nanoparticles, and the mice did not show any significant weight loss. After the treatment period, the mice are euthanized and their blood is collected for routine analysis. As observed, the hematological toxicity (dose-limited toxicity) of nanoparticles is extremely low or even zero. These results demonstrate that amino anti-tumor drug nanoparticles administered by intravenous injection are a drug carrier system of low blood toxicity.

The above-mentioned embodiments only express the specific implementation of the present invention, and are descried more specifically and in detail, but the embodiments shall not be construed to limit the scope of the present invention. It should be note that for those ordinary skilled in the art, without departing from the concept of the present invention, several modifications and improvements can be made, which fall within the scope of the present invention.

## Claims

1. A heparin nano drug carrier system loaded with an amino anti-tumor drug, **characterized in that** this drug carrier system is a conjugate formed by loading the amino anti-tumor drug onto a PEGylated heparin molecule; and a specific structure is as follows: 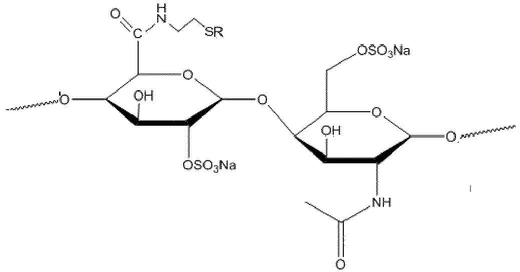
where: R is a PEG group and a D group;
the PEG group is: 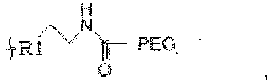 and this group is an acyl group connected with a hydroxyl group at the end of PEG via an ester bond,
where: R1 is 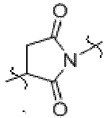 or -S-;
the structure of the D group is: 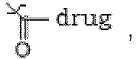 and this group is an acyl group connected with an amino group in the drug molecule via an amido bond.

2. The heparin nano drug carrier system loaded with the amino anti-tumor drug according to claim 1, **characterized in that** polyethylene glycol (PEG) is mPEG2000.

3. The heparin nano drug carrier system loaded with the amino anti-tumor drug according to claim 1, **characterized in that** an amino group in the amino anti-tumor drug is a primary amino group or a secondary amino group.

4. The heparin nano drug carrier system loaded with the amino anti-tumor drug according to claim 1 or 3, **characterized in that** the amino anti-tumor drug comprises:
daunorubicin, lenalidomide, lapatinib, procarbazine, mitoxantrone, clofarabine or nelarabine.

5. A preparation method of the heparin nano drug carrier system loaded with the amino anti-tumor drug according to claim 1, **characterized by** comprising the following synthesis scheme:
(1) preparing a PEG derivative; 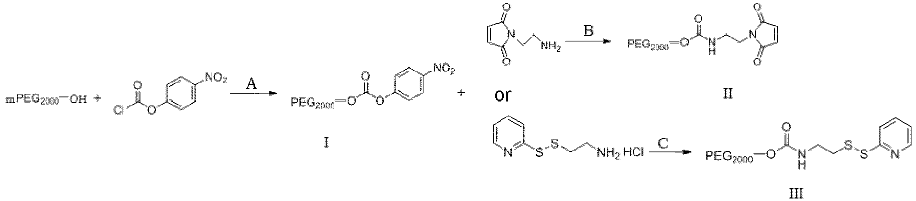
(2) preparing an intermediate A; 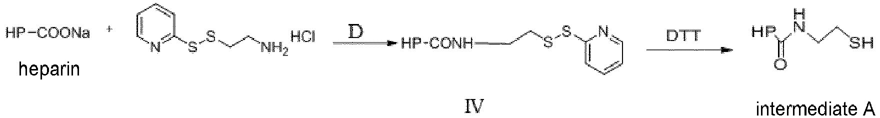
(3) reacting the intermediate A with the prepared PEG derivative to obtain a PEGylated heparin; and
(4) reacting the PEGylated heparin with 4-nitrophenyl chloroformate, then adding the amino anti-tumor drug into the reaction system, to obtain the heparin nano drug carrier system loaded with the drug.

6. The preparation method of the heparin nano drug carrier system loaded with the amino anti-tumor drug according to claim 5, **characterized in that** in the reaction A and reaction B of step (1), a catalyst DIEA is added.

7. The preparation method of the heparin nano drug carrier system loaded with the amino anti-tumor drug according to claim 5, **characterized in that** in the reaction C of step (1), catalysts DIEA and DMAP are added.

8. The preparation method of the heparin nano drug carrier system loaded with the amino anti-tumor drug according to claim 5, **characterized in that** in the reaction D, enoxaparin sodium is dissolved in a MeS buffer solution and activated by adding DMTMM, then S-(2-aminoethylthio)-2-thiopyridine that is dissolved in the MeS buffer solution is added dropwise to the system for reaction, to obtain the intermediate A.

9. The preparation method of the heparin nano drug carrier system loaded with the amino anti-tumor drug according to claim 8, **characterized in that** a preparation method of the MeS buffer comprises: weighing a quinoline-8-sulfonic acid and dissolving the same in purified water, adding a sodium hydroxide solution dropwise to adjust the pH to 5.5, and making a metered volume, to obtain the MeS buffer solution.

10. The preparation method of the heparin nano drug carrier system loaded with the amino anti-tumor drug according to claim 5, **characterized in that** in the reaction of step (3), the intermediate A reacts with a polymer III, by addition of triethylamine as a catalyst.

11. The heparin nano drug carrier system loaded with the amino anti-tumor drug according to claim 1, **characterized in that** a drug loading capacity of the nano drug carrier system is 4 wt%-15 wt%.
